# DEMANDE DE BREVET EUROPEEN

(11) **EP 4 491 111 A1**
(43) Date de publication de la demande: **15.01.2025**
(21) Numéro de dépôt: 23306208.2
(22) Date de dépôt: 13.07.2023
(51) Int. Cl.: A61B 5/11

(54) **PROCEDE ET DISPOSITIF POUR LE SUIVI D'UN INDIVIDU ATTEINT D'UNE MALADIE NEURODEGENERATIVE**

(71) Demandeur: Diampark, 92370 Chaville (FR)
(72) Inventeur: DALILI, Djamchid, 92370 CHAVILLE (FR)
(74) Mandataire: Icosa

(57) **Abrégé**

L'invention concerne un dispositif pour surveiller une évolution temporelle, pendant une période temporelle [tᵢ,t_{f}], d'un état de santé d'un individu atteint d'une maladie neurodégénérative.

## Description

### DOMAINE DE L'INVENTION

La présente invention concerne un procédé mis en oeuvre par ordinateur pour surveiller une évolution temporelle, pendant une période temporelle, d'un état de santé d'un individu atteint d'une maladie neurodégénérative.

### ÉTAT DE LA TECHNIQUE

Les maladies neurodégénératives sont des pathologies chroniques, qui s'installent progressivement et affectent le système nerveux central en provoquant la dégénérescence de certains neurones du système nerveux central. On dénombre plus d'une centaine de maladies neurodégénératives, caractérisées par le groupe de neurones atteints par la dégénérescence.

Les troubles neurodégénératifs engendrent des dysfonctionnements de l'équilibre, des mouvements, de la parole, ou encore de la respiration. Par exemple, les dysfonctionnements des mouvements peuvent se traduire par des tremblements au repos ou une rigidité musculaire.

A l'heure actuelle, les troubles neurodégénératifs ne peuvent pas être guéris. La prise en charge de patients atteints vise à freiner la progression de la maladie et à soulager les symptômes pour prolonger l'autonomie des patients. Ainsi, des traitements médicamenteux pris sur le long terme permettent de soulager les symptômes de la maladie.

Il pourrait s'avérer avantageux de tirer parti de données du quotidien de patients atteints d'une maladie neurodégénérative et prenant un traitement médicamenteux, pour le suivi des effets de ce traitement. En effet, la « vraie vie » (en anglais « real-world evidence », ou RWE) permet d'obtenir des informations utiles complémentaires aux données d'essais cliniques, avec le potentiel de combles des lacunes des connaissances, notamment concernant l'utilisation de médicaments, dans la pratique clinique de routine.

L'invention s'inscrit dans ce contexte.

### RÉSUMÉ

Un premier aspect de l'invention concerne un dispositif pour surveiller une évolution temporelle, pendant une période temporelle [ti,tf], d'un état de santé d'un individu atteint d'une maladie neurodégénérative, comprenant :
- au moins une interface d'entrée configurée pour :
   - recevoir au moins une séquence temporelle de mesures d'un biomarqueur représentatif d'un symptôme de ladite maladie neurodégénérative, lesdites mesures étant acquises par au moins un capteur ;
   - recevoir un diagramme de référence prédéterminé ;
- au moins une unité de traitement configurée pour :
   - calculer, pour chaque mesure, un couple de valeurs formé d'une première valeur et d'une deuxième valeur,
   - calculer, pour chaque couple de valeurs associé à une mesure de ladite séquence temporelle, une position correspondante sur ledit diagramme de référence prédéterminé,
   - déterminer un diagramme de mesure comprenant une superposition du diagramme de référence prédéterminé et desdites positions correspondantes,
- au moins une interface de sortie configurée pour fournir le diagramme de mesure, l'ensemble des positions correspondantes calculées formant sur le diagramme de mesure une trajectoire représentative de l'évolution temporelle dudit état de santé pendant ladite période temporelle [ti,tf].

Ainsi, avantageusement, l'ensemble des positions forment une trajectoire sur le diagramme de mesure permet d'aider un utilisateur (i. e. ; personnel de santé) à tirer des conclusions sur l'évolution temporelle de l'état de santé de l'individu pendant la période temporelle [ti,tf], telle qu'une évolution favorable, ou une inadéquation d'un traitement pris par l'individu et par exemple d'ajuster en conséquence le traitement.

Dans un ou plusieurs modes de réalisation, le diagramme de référence prédéterminé comprend au moins un moyen d'identification d'au moins une portion dudit diagramme de mesure, ledit moyen peut-être pour exemple une ou plusieurs lignes verticales ou horizontales permettant un quadrillage du diagramme de référence. Plus particulièrement, la localisation de certaines positions correspondantes de mesures en certaines portions du diagramme de mesure peut être interpréter grâce à la signification particulière de ces portions.

Dans un ou plusieurs modes de réalisation, la maladie neurodégénérative est une maladie pour laquelle le symptôme est un symptôme principal ou secondaire consistant en des tremblements d'une partie du corps, comme la maladie de Parkinson, la sclérose en plaques, ou la maladie de Huntington.

Dans un ou plusieurs modes de réalisation :
- la maladie neurodégénérative est la maladie de Parkinson, la sclérose en plaques, ou la maladie de Huntington, et le symptôme est un tremblement d'une main de l'individu,
- le diagramme de référence prédéterminé est un diagramme bidimensionnel comprenant un ensemble de points de référence de densité donnée, avec un axe d'abscisses représentant une fréquence de tremblement, et un axe d'ordonnées représentant une intensité de tremblement,
- pour chaque mesure de la séquence temporelle, la première valeur est une fréquence de tremblement et la deuxième valeur est une intensité de tremblement normalisée.

Dans un ou plusieurs modes de réalisation, le diagramme de référence prédéterminé comprend une ligne de référence fixe, nommée ligne de crête, au voisinage de laquelle sont localisées les positions correspondantes calculées.

Dans un ou plusieurs modes de réalisation, le diagramme de référence prédéterminé a été préalablement obtenu par une campagne de mesures temporelles dudit biomarqueur sur un sujet de référence atteint de ladite maladie neurodégénérative pendant un intervalle de temps prédéfini.

Dans un ou plusieurs modes de réalisation, le diagramme de référence prédéterminé comprend, dans un premier voisinage de la ligne de crête, une première zone représentative d'un premier état de santé, et, dans un deuxième voisinage de la ligne de crête distinct du premier voisinage, une deuxième zone représentative d'un deuxième état de santé, le premier état de santé étant dégradé par rapport au deuxième état de santé. Ainsi, il pourra être automatiquement déduit de la présence de positions correspondantes de mesures dans la première zone ou la deuxième zone du diagramme de référence si l'état de santé correspondant de l'individu est dégradé ou non.

Dans un ou plusieurs modes de réalisation, le capteur est une centrale inertielle intégrée dans une montre connectée ou dans un ordiphone.

Dans un ou plusieurs modes de réalisation, le capteur est un microphone et le biomarqueur est déductible d'un enregistrement de la voix de l'individu.

Dans un ou plusieurs modes de réalisation, le capteur est un capteur d'images et le biomarqueur est déductible, par exemple à partir de clignements ou d'autres mouvements des yeux de l'individu.

Dans un ou plusieurs modes de réalisation, le capteur est un écran tactile et le biomarqueur est un biomarqueur de justesse représentatif de la justesse du geste lors de la calligraphie.

Dans un ou plusieurs modes de réalisation, le biomarqueur est un biomarqueur parmi le taper des doigts (en anglais « finger tapping »), le taper des orteils (en anglais « toes tapping », le taper des pieds (en anglais « foot tapping »).

Un autre aspect de l'invention porte sur un procédé mis en oeuvre par ordinateur pour surveiller une évolution temporelle, pendant une période temporelle [ti,tf], d'un état de santé d'un individu atteint d'une maladie neurodégénérative, comprenant :
- recevoir au moins une séquence temporelle de mesures d'un biomarqueur représentatif d'un symptôme de ladite maladie neurodégénérative pendant ladite période temporelle [ti,tf], lesdites mesures étant acquises par au moins un capteur,
- recevoir un diagramme de référence prédéterminé,
- calculer, pour chaque mesure, un couple de valeurs formé d'une première valeur et d'une deuxième valeur,
- calculer, pour chaque couple de valeurs associé à une mesure de la séquence temporelle, une position correspondante sur ledit diagramme de référence prédéterminé,
- déterminer un diagramme de mesure comprenant une superposition du diagramme de référence prédéterminé et desdites positions correspondantes,
l'ensemble des positions correspondantes calculées formant sur le diagramme de mesure une trajectoire représentative de l'évolution temporelle dudit état de santé pendant ladite période temporelle [ti,tf].

Avantageusement, le procédé est mis en oeuvre par le dispositif pour surveiller une évolution temporelle, pendant une période temporelle [ti,tf], d'un état de santé d'un individu atteint d'une maladie neurodégénérative précédemment décrit.

Dans un ou plusieurs modes de réalisation :
- la maladie neurodégénérative est la maladie de Parkinson et le symptôme est un tremblement d'une main de l'individu,
- le diagramme de référence est un diagramme bidimensionnel comprenant un ensemble de points de référence de densité donnée, avec un axe d'abscisses représentant une fréquence de tremblement, et un axe d'ordonnées représentant une intensité de tremblement,
- pour chaque mesure de la séquence temporelle, la première valeur est une fréquence de tremblement et la deuxième valeur est une intensité de tremblement normalisée.

Dans un ou plusieurs modes de réalisation, le diagramme de référence comprend une ligne de référence fixe, nommée ligne de crête, au voisinage de laquelle sont localisées les positions correspondantes calculées.

Dans un ou plusieurs modes de réalisation, le diagramme de référence a été obtenu par une campagne de mesures temporelles dudit biomarqueur sur un sujet de référence atteint de ladite maladie neurodégénérative pendant un intervalle de temps prédéfini.

Dans un ou plusieurs modes de réalisation, le diagramme de référence comprend, dans un premier voisinage de la ligne de crête, une première zone représentative d'un premier état de santé, et, dans un deuxième voisinage de la ligne de crête distinct du premier voisinage, une deuxième zone représentative d'un deuxième état de santé, le premier état de santé étant dégradé par rapport au deuxième état de santé.

Avantageusement, le capteur est une centrale inertielle intégrée dans une montre connectée ou dans un ordiphone.

Dans un ou plusieurs modes de réalisation, le procédé comprend en outre :
- afficher ledit diagramme de mesure,
- déterminer un premier ensemble de positions parmi l'ensemble des positions calculées compris dans la première zone,
- identifier, sur la base dudit premier ensemble de positions, un ensemble d'instants nommés instants dégradés, pendant la période temporelle [ti,tf], durant lesquels l'individu était dans le premier état de santé.

Ainsi, par l'identification des instants dégradés, le procédé selon l'invention permet de tirer des conclusions sur l'adéquation d'un traitement pris par l'individu et d'ajuster en conséquence ce traitement.

Dans un ou plusieurs modes de réalisation, le procédé comprend en outre :
- afficher ledit diagramme de mesure,
- déterminer un premier ensemble de positions parmi l'ensemble des positions calculées compris dans la première zone,
- déterminer un deuxième ensemble de positions parmi l'ensemble des positions calculées compris dans la deuxième zone,
- déduire du premier ensemble de positions et du deuxième ensemble de positions un état de santé global du patient pendant la période temporelle [ti,tf].

Un autre aspect de l'invention porte sur un produit programme informatique comportant des instructions pour mettre en oeuvre des étapes du procédé précédemment décrit, lorsque ce programme est exécuté par un processeur.

Encore un autre aspect de l'invention porte sur un support d'enregistrement lisible par ordinateur non-transitoire comprenant les instructions qui lorsqu'elles sont exécutées par un ordinateur, conduisent celui-ci à mettre en oeuvre des étapes du procédé décrit ci-dessus.

Encore un autre aspect de l'invention porte sur un dispositif pour obtenir un diagramme de référence d'un individu de référence atteint d'une maladie neurodégénérative dont un symptôme est un tremblement d'une partie du corps, le dispositif comprenant :
- au moins une interface d'entrée configurée pour :
   - recevoir au moins une séquence temporelle de mesures d'un biomarqueur représentatif du tremblement de la partie du corps, lesdites mesures étant acquises par au moins un capteur ;
- au moins une unité de traitement configurée pour :
   - recevoir un diagramme initial bidimensionnel comprenant un axe horizontal correspondant aux fréquences temporelles de tremblement et un axe vertical correspondant aux intensités de tremblement normalisées,
   - calculer, pour chaque mesure, un couple de valeurs formé d'une première valeur et d'une deuxième valeur,
   - calculer, pour chaque couple de valeurs associé à une mesure de ladite séquence temporelle, une position correspondante sur ledit diagramme initial bidimensionnel,
   - marquer chaque position correspondante sur ledit diagramme initial bidimensionnel,
   l'ensemble formé par le diagramme initial bidimensionnel et les positions correspondantes constituant le diagramme de référence,
- au moins une interface de sortie configurée pour fournir le diagramme de référence.

### DÉFINITIONS

Dans la présente invention, les termes ci-dessous sont définis de la manière suivante : le terme « processeur » ne doit pas être interprété comme étant limité au matériel informatique (« hardware ») capable d'exécuter un logiciel, et se réfère de manière générale à un dispositif de traitement, qui peut par exemple inclure un ordinateur, un microprocesseur, un circuit intégré ou un dispositif logique programmable (« programmable logic device », PLD). Le processeur peut également englober un ou plusieurs processeurs graphique (GPU), qu'ils soient exploités pour l'infographie et le traitement d'images ou d'autres fonctions. En outre, les instructions et/ou les données permettant d'exécuter les fonctionnalités associées et/ou résultantes peuvent être stockées sur tout support lisible par le processeur tel que, par exemple, un circuit intégré, un disque dur, un CD (Compact Disc), un disque optique tel qu'un DVD (Digital Versatile Disc), une RAM (Random-Access Memory) ou une ROM (Read-Only Memory). Les instructions peuvent notamment être stockées dans du matériel informatique, des logiciels, des microprogrammes (« firmware ») ou dans toute combinaison de ceux-ci.

« Centrale inertielle » concerne des instruments comprenant six capteurs, desquels trois gyromètres mesurant les trois composantes du vecteur vitesse angulaire, et trois accéléromètres mesurant les trois composantes du vecteur force spécifique.

### DESCRIPTION DES FIGURES

La figure 1 est un diagramme schématique montrant les composants d'un exemple d'un dispositif pour surveiller une évolution temporelle, pendant une période temporelle [tᵢ,t_{f}], d'un état de santé d'un individu atteint d'une maladie neurodégénérative selon plusieurs modes de réalisation.
   La représente un exemple d'étapes mises en oeuvre pour la réalisation d'une phase de calibration, selon un ou plusieurs modes de réalisation.
La figure 2 représente un exemple de diagramme de référence prédéterminé pouvant être utilisé avec le dispositif représenté à la figure 1.
La figure 3 représente un exemple d'étapes mises en oeuvre pour la réalisation d'un procédé mis en oeuvre par ordinateur pour surveiller une évolution temporelle, pendant une période temporelle [ti,tf], d'un état de santé d'un individu atteint d'une maladie neurodégénérative, selon un ou plusieurs modes de réalisation.
La figure 4 représente un exemple de diagramme de mesure pouvant être obtenu avec le dispositif représenté à la figure 1.
La figure 5 représente un autre exemple diagramme de mesure pouvant être obtenu avec le dispositif représenté à la figure 1.

### DESCRIPTION DÉTAILLÉE

Un premier aspect de l'invention porte sur un dispositif pour surveiller une évolution temporelle, pendant une période temporelle [tᵢ,t_{f}], d'un état de santé d'un individu atteint d'une maladie neurodégénérative, telle que la maladie de Parkinson, la sclérose en plaques ou la maladie de Huntington.

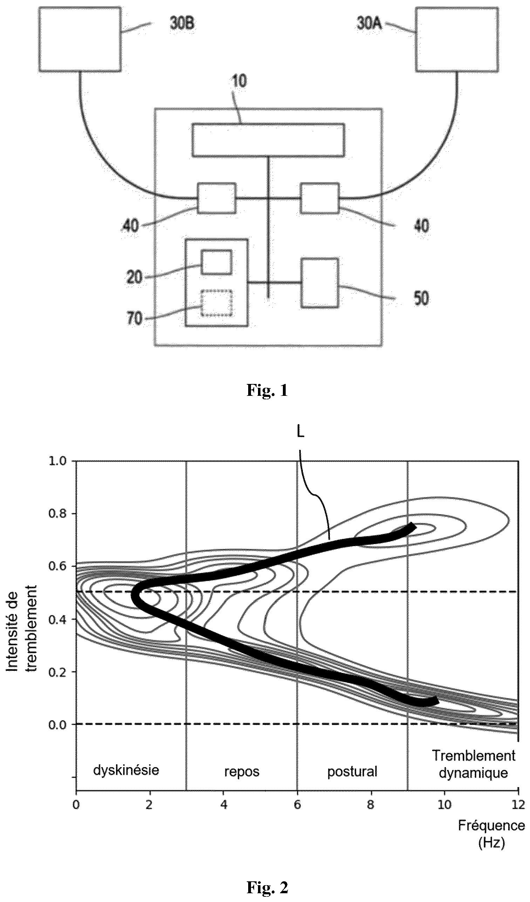

La figure 1 est un diagramme schématique montrant les composants d'un exemple d'un tel dispositif 1.

Le dispositif 1 peut être mis en oeuvre en tant que dispositif matériel unique, par exemple sous la forme d'un ordinateur personnel (PC) de bureau, d'un ordinateur portable, d'un assistant numérique personnel (PDA), d'un ordiphone, d'une montre connectée, d'un serveur, d'une console ou peut être mis en oeuvre sur des dispositifs matériels interconnectés séparés interconnectés par un ou plusieurs liens de communication, avec des segments câblés et/ou sans fil. Le dispositif 1 peut par exemple être en communication avec un ou plusieurs systèmes informatiques en nuage, un ou plusieurs serveurs ou dispositifs distants pour mettre en oeuvre les fonctions décrites dans le présent document pour le dispositif concerné. Le dispositif 1 peut également être mis en oeuvre lui-même en tant que système informatique en nuage.

Comme représenté schématiquement sur la figure 1, le dispositif 1 peut comprendre au moins un processeur 10 configuré pour accéder à au moins une mémoire 20 comprenant un code de programme informatique 70. Le code de programme informatique peut comprendre des instructions configurées pour amener le système de détermination 1 à exécuter une ou plusieurs ou toutes les étapes du procédé de détermination 100 pour surveiller une évolution temporelle, pendant une période temporelle [tᵢ,t_{f}], d'un état de santé d'un individu décrit précédemment.

Le processeur 10 peut ainsi être configuré pour stocker, lire, charger, interpréter, exécuter et/ou traiter autrement le code de programme informatique 70 stocké dans la mémoire 20 de sorte que, lorsque les instructions codées dans le code de programme informatique sont exécutées par le au moins un processeur 10, le dispositif 1 exécute une ou plusieurs étapes du procédé 100 décrit ici.

Le processeur 10 peut être n'importe quel microprocesseur, microcontrôleur, circuit intégré ou unité centrale de traitement (CPU) approprié comprenant au moins un processeur ou noyau de traitement basé sur le matériel.

La mémoire 20 peut comprendre une mémoire vive (RAM), une mémoire cache, une mémoire non volatile, une mémoire de sauvegarde (par exemple, des mémoires programmables ou flash), une mémoire morte (ROM), un disque dur (HDD), un lecteur à état solide (SSD) ou toute combinaison de ceux-ci. La ROM de la mémoire 20 peut être configurée pour stocker, entre autres, un système d'exploitation du système S et/ou un ou plusieurs codes de programmes informatiques d'une ou plusieurs applications logicielles. La RAM de la mémoire 20 peut être utilisée par le processeur 10 pour le stockage temporaire de données.

Le dispositif 1 peut en outre comprendre une ou plusieurs interfaces de communication 40 (par exemple, des interfaces de réseau pour l'accès à un réseau câblé / sans fil, y compris une interface Ethernet, une interface WIFI, des interfaces USB, etc. Le dispositif 1 peut comprendre d'autres matériels associés tels que des interfaces utilisateurs comme une interface de rendu visuel 2D 30A, ou des interfaces haptiques 30B, ou toutes autres interfaces (par exemple clavier, souris, écran d'affichage, etc) connectées via une ou plusieurs interfaces de communication appropriées 40 avec le processeur. Le dispositif 1 peut également comprendre un lecteur de support 50 pour lire un support de stockage externe lisible par ordinateur. Le processeur 10 est connecté à chacun des autres composants afin d'en commander le fonctionnement.

Le dispositif 1 est configuré pour mettre en oeuvre un procédé 100 mis en oeuvre par ordinateur pour aider à la surveillance de l'évolution temporelle, pendant une période temporelle [ti,tf], d'un état de santé d'un individu atteint d'une maladie neurodégénérative, sur la base d'une séquence temporelle de mesures d'un biomarqueur représentatif d'un symptôme de la maladie neurodégénérative, lesdites mesures étant acquises par au moins un capteur.

Par exemple, le symptôme peut être un symptôme principal ou secondaire tels que des tremblements d'une portion du corps de l'individu, tel que dans le cas de la maladie de Parkinson, la sclérose en plaques, ou la maladie de Huntington.

Dans un ou plusieurs modes de réalisation, le capteur est un microphone et le biomarqueur est déductible d'un enregistrement de la voix de l'individu.

Dans un ou plusieurs modes de réalisation, le capteur est un capteur d'images et le biomarqueur est déductible par exemple de clignements ou de mouvements des yeux de l'individu.

Dans un ou plusieurs modes de réalisation, le capteur est un écran tactile et le biomarqueur est un biomarqueur de justesse représentatif de la justesse du geste lors de la calligraphie.

Dans un ou plusieurs modes de réalisation, le biomarqueur est un biomarqueur parmi le « finger tapping », le « toe tapping », le « foot tapping ».

Le procédé 100 est basé sur l'utilisation d'un diagramme de référence prédéterminé obtenu préalablement sur la base de mesures temporelles d'un biomarqueur d'un sujet de référence atteint de la maladie neurodégénérative (e.g., maladie de Parkinson) obtenues lors d'une campagne de mesures menée pendant un intervalle de temps prédéfini. Par exemple, le biomarqueur est représentatif du symptôme de tremblement des mains caractéristique de la maladie de Parkinson, mais qui est présent aussi dans la sclérose en plaques, ou la maladie de Huntington. Typiquement, ce biomarqueur est mesuré au moyen d'au moins un capteur, notamment au gyroscope, un accéléromètre, une centrale inertielle ou tous capteur connue par l'homme du métier configuré pour obtenir une mesure représentative des mouvements d'au moins une portion du corps du patient (e.g., tremblement d'une main). Par exemple, ce biomarqueur peut être mesuré au moyen d'une montre connectée portée par le sujet de référence, ou d'un ordiphone porté par la main du sujet de référence de façon récurrente, la montre connectée ou l'ordiphone comprenant un capteur de type centrale inertielle intégrée. Par exemple, la campagne de mesures dure pendant une semaine à plusieurs mois et un minimum de 100 mesures sont recueillies pour la construction du diagramme de référence prédéterminé.

La figure 2 montre un exemple d'un diagramme de référence, dans le cas de la maladie de Parkinson, prédéterminé correspondant à une campagne de mesures sur un sujet de référence à partir de 86000 mesures prises pendant un intervalle de temps prédéfini de trois mois. Comme montré dans cet exemple, le diagramme de référence est un diagramme bidimensionnel comprenant un ensemble de points de référence de densité donnée, avec un axe d'abscisses représentant une fréquence de tremblement en Hertz, et un axe d'ordonnées représentant une intensité de tremblement normalisée (i.e., adimensionnelle).

On décrit ci-après l'obtention de la valeur de l'intensité de tremblement normalisée correspondant à l'une des mesures prises pendant l'intervalle de temps prédéfini. Le capteur fournit des données brutes correspondant aux accélérations suivant trois directions perpendiculaires x, y et z. Une analyse en fréquence des données brutes est réalisée à intervalles réguliers, par exemple, toutes les 5 secondes. Par exemple, l'analyse en fréquence peut consister en un calcul de transformée de Fourier, afin d'obtenir le spectre en fréquence des données brutes.

Dans un mode de réalisation, l'intensité de tremblement normalisée qui est utilisée pour le diagramme de référence prédéterminé est calculée sur la base de la puissance crête du spectre en fréquence (« peak power »).

Dans un autre mode de réalisation, l'intensité de tremblement normalisée qui est utilisée pour le diagramme de référence prédéterminé est calculée sur la base d'une puissance fenêtrée (« windowed power » du spectre en fréquence). Par exemple, la puissance fenêtrée peut être calculée par intégration du spectre sur la bande [3, 12 Hz] ou sur la bande [3, 9 Hz].

Dans encore un autre mode de réalisation, l'intensité de tremblement normalisée qui est utilisée pour le diagramme de référence prédéterminé est calculée sur la base de la puissance totale du spectre en fréquence. En pratique, la puissance totale peut être calculée par intégration du spectre sur une bande finie telle que la bande [0, 50 Hz].

Par exemple, l'intensité de tremblement normalisée est une variable composite de la fréquence de tremblement et d'une puissance calculée sur la base du spectre en fréquence, par exemple une puissance crête, une puissance fenêtrée ou une puissance totale. Cette variable composite peut être obtenue par l'implémentation d'un modèle d'apprentissage automatique configuré notamment pour recevoir en entrée le spectre en fréquence complet des tremblements (i.e., aucune bande de fréquence n'est supprimée). Ce mode de réalisation, il est particulièrement avantageux car il permet de compresser l'information concernant toutes les fréquences de tremblement dans une variable composite encodée sans pour autant perdre d'information.

Chaque point de référence du diagramme de référence prédéterminé représente une densité ou en d'autres termes un nombre d'occurrences d'une intensité de tremblement donnée à une certaine fréquence, pendant l'intervalle de temps de la campagne de mesures. L'ensemble des valeurs de l'intensité de tremblement et fréquences issues mesures prises est utilisé pour déterminer des courbes de niveau, comme représenté dans diagramme de référence prédéterminé en figure 2. Chaque point de référence d'une même courbe de niveau présente la même densité.

Le diagramme de référence prédéterminé est comparable à une photographie longue exposition où sont accumulées des mesures représentatives du biomarqueur pendant l'intervalle de temps de la campagne de mesures, où se sont accumulées les mesures de fréquence et d'intensité de tremblement normalisée, représenté par les courbes de niveau.

Par rapport à l'exemple en figure 2 concernant la maladie de Parkinson, trois bandes de fréquence de tremblement sont remarquables sur le diagramme de référence prédéterminé, et repérables au niveau des îlots des courbes de niveau de plus forte densité : autour de 1,9 Hz, bande correspondant à la dyskinésie ; autour de 5 Hz et autour de 9 - 10 Hz, bandes correspondant aux tremblements dynamiques. Ces trois bandes de fréquence correspondent aux fréquences de contraction des muscles caractéristiques de sujets atteints de la maladie de Parkinson.

Dans cet exemple, une ligne de référence fixe L, dénommée ci-après ligne de crête, est définie et représentée sur le diagramme de référence prédéterminé. Par exemple, la ligne de crête est une ligne d'interpolation passant par les maxima des îlots du diagramme de référence prédéterminé, comme représenté en figure 2.

Les inventeurs de la présente demande ont découvert que, lorsque des mesures d'un biomarqueur représentatif de tremblements de la main d'un individu atteint de la maladie de Parkinson sont prises, enregistrées sous la forme de points ayant pour coordonnées la fréquence du tremblement et l'intensité normalisée du tremblement, ces points se situaient au voisinage de la ligne de crête du diagramme de référence prédéterminé. Autrement dit, les mesures d'un tel biomarqueur se déplacent globalement autour de la ligne de crête L. La position du point correspondant à la mesure du biomarqueur par rapport à la ligne de crête L dépend de l'état de santé de l'individu, comme cela va être détaillé par la suite.

Aussi, il est à noter que la zone du diagramme de référence prédéterminé où les intensités normalisées de tremblement sont supérieures à 0.5 correspond à des tremblements perceptibles à l' oeil, tout en étant mesurables par un capteur. Au contraire, la zone du diagramme de référence où les intensités normalisées de tremblement sont inférieures à 0.5 correspond à des tremblements imperceptibles à l'oeil, mais mesurables par un capteur. Les portions du diagramme de référence prédéterminé correspondant aux valeurs supérieures ou inférieures à 0.5 sont identifiées par un moyen de visualisation, par exemple une ligne en pointillés comme en figure 2.

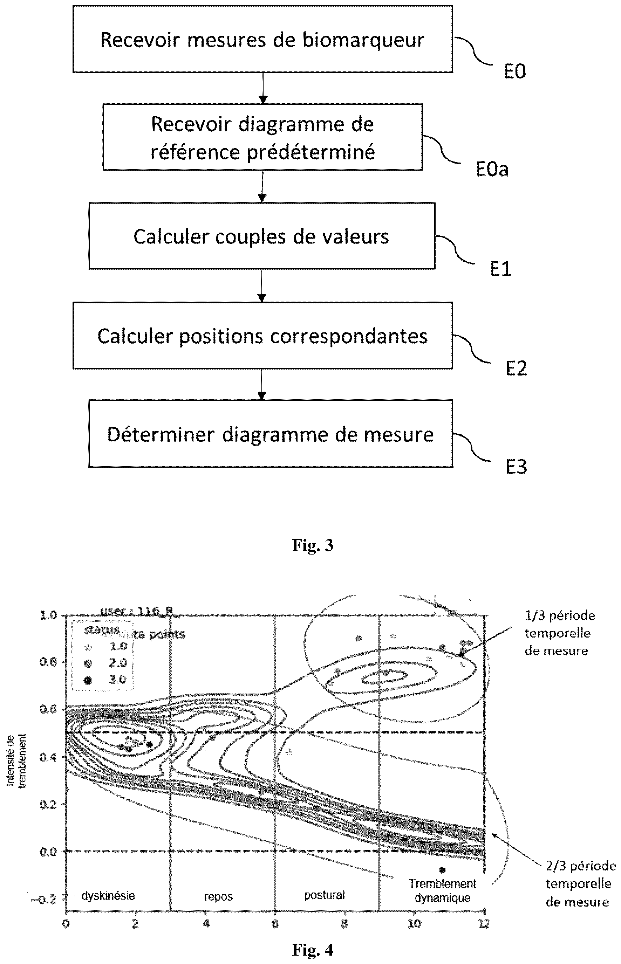

Le procédé 100 mis en oeuvre par ordinateur pour surveiller une évolution temporelle, pendant une période temporelle [ti,tf], d'un état de santé d'un individu atteint de la maladie de neurodégénérative est décrit ci-dessous. La figure 3 montre un exemple d'ensemble d'étapes pouvant être effectuées pour mettre en oeuvre le procédé 100.

On suppose que la fréquence et l'amplitude des tremblements d'une partie du corps de l'individu (e.g., des mains de l'individu) sont mesurées périodiquement, ou en continu, ou de manière éparse au moyen d'au moins un capteur de mesure des tremblements (e.g., type centrale inertielle, camera, microphone etc.). Par exemple, l'individu porte au poignet une montre connectée intégrant une centrale inertielle. Alternativement ou en plus, les mesures peuvent être prises lorsque l'individu tient son ordiphone personnel, l'ordiphone intégrant une centrale inertielle.

Lors d'une étape E0, une séquence temporelle de mesures du biomarqueur prises pendant la période temporelle [tᵢ,t_{f}] est collectée. Optionnellement, la séquence temporelle est stockée dans une mémoire du dispositif 1. Alternativement, la séquence temporelle peut être traitée à la volée par le circuit du dispositif 1.

Chaque mesure provient de données brutes détectées par le(s) capteur(s). Dans le cas, de la centrale inertielle, les données brutes sont par exemple composées de trois valeurs d'accélération selon trois axes indépendants. Les données brutes sont acquises à une fréquence de mesure donnée. Par exemple, les trois valeurs d'accélération sont reçues toutes les 10 millisecondes.

Préalablement, ou avant la mise en oeuvre des étapes E2 et E3 qui vont être décrites par la suite, dans une étape E0a, le dispositif 1 reçoit un diagramme de référence prédéterminé tel que celui représenté sur la figure 2.

Dans une étape E1, les données brutes sont transformées en couples de valeurs intermédiaires formés d'une fréquence et d'une amplitude. Puis, les couples de valeurs intermédiaires sont transformés en couples de valeurs formés d'une fréquence de tremblement et d'une intensité normalisée de tremblement. La valeur d'intensité normalisée de tremblement de la mesure est comprise entre 0 et 1. Par exemple, pour normaliser l'amplitude du signal, celle-ci peut être divisée par l'amplitude maximale des tremblements du sujet de référence dont les mesures du biomarqueur ont été utilisées pour créer le diagramme de référence prédéterminé. Dans un autre exemple, l'intensité de tremblement normalisée peut être calculée comme variable composite de la fréquence de tremblement et d'une puissance calculée à partir du spectre en fréquence des données brutes, telle qu'une puissance crête, une puissance fenêtrée ou une puissance totale et en utilisant un modèle d'apprentissage automatique.

Lors d'une étape E2, l'au moins un processeur 10 calcule une position correspondante sur le diagramme de référence prédéterminé pour chaque mesure de la séquence temporelle de mesures du biomarqueur. Plus précisément, les coordonnées d'une position correspondante d'une mesure sont la fréquence de tremblement de la mesure et l'intensité normalisée de tremblement de la mesure.

Lors d'une étape E3, le au moins un processeur 10 détermine un diagramme de mesure. Le diagramme de mesure comprend une superposition du diagramme de référence prédéterminé et des positions correspondantes.

Avantageusement, lorsque le dispositif 1 comprend une interface de rendu visuel 30A, le diagramme de mesure peut être visualisé sur cette interface de rendu visuel 30A.

La figure 3 montre un exemple de diagramme de mesure. Les positions correspondantes des mesures sont représentées par des cercles remplis. Tout autre type de symbole peut être utilisé pour représenter les positions correspondantes des mesures. On peut observer que ces positions se situent dans le voisinage de la ligne de crête.L'analyse d'un tel diagramme de mesure permet de tirer des conclusions sur l'état de santé de l'individu dont on a pris les mesures au cours de la période temporelle [tᵢ,t_{f}].

Un premier exemple d'analyse peut être donné en référence à la figure 4. La figure 4 correspond au diagramme de mesure montré à la figure 3, où une première zone et une deuxième zone ont été délimitées. Les mesures prises sur le patient sont réparties dans la première zone et la deuxième zone.

La première zone, située dans la moitié inférieure du diagramme de référence, c'est-à-dire correspondant à des mesures présentant une fréquence de tremblement s'échelonnant de 0 à 12 Hz, avec une intensité normalisée de tremblement inférieure à 0.5, correspond à un état de santé stable. Ainsi, si des positions correspondantes de mesure se situent dans la première zone, ces positions correspondent à un état de santé stable. Ceci peut correspondre à des instants où l'individu, prenant son traitement médicamenteux contre la maladie, présente une bonne réaction à ce traitement et que ce dernier fait effet. Dans l'exemple spécifique de la figure 4, il peut être conclu que le traitement médicamenteux pris par l'individu fait effet sur une durée cumulée d'environ deux tiers de la période temporelle de mesure.

La deuxième zone, située dans le cadrant supérieur droit du diagramme de référence, c'est à -dire correspondant à mesures présentant une fréquence de tremblement comprise entre 8 et 12 Hz et une intensité normalisée de tremblement supérieure à 0.5, correspond à des états de santé d'alerte, c'est à-dire où l'individu présente de forts symptômes et où son état est dégradé. Ainsi, si des positions correspondantes de mesure se situent dans la deuxième zone, ces positions correspondent à de tels états de santé d'alerte. Ceci peut correspondre à des instants où l'individu échappe à son traitement médicamenteux et se trouve dans un état de santé dégradé par rapport aux instants de mesures situées dans la première zone. Dans l'exemple spécifique de la figure 4, il peut être conclu que l'individu échappe au traitement sur une durée cumulée d'environ un tiers de la période temporelle de mesure.

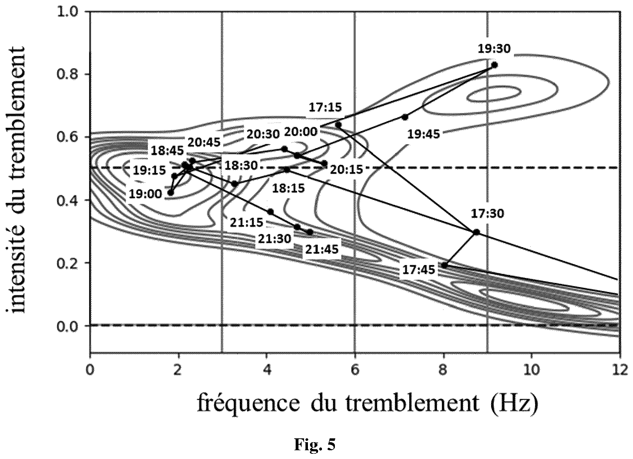

Dans un autre exemple, il est également possible de suivre, grâce à la trajectoire formée par l'ensemble des points de mesure sur le diagramme de référence, et tel qu'illustré sur la figure 5 les effets du traitement médicamenteux sur l'individu et/ou l'historique des prises de traitement par l'individu au cours de la période temporelle [tᵢ,t_{f}].

En observant la figure 5, on peut tirer les conclusions suivantes. A 17h15, le traitement médicamenteux commence à faire effet. A 17h30, il n'y a plus de tremblement visible à l'oeil. Vers 19h00, on observe la fin de l'effet du traitement médicamenteux. A 19h30, on se situe dans une zone où il existe beaucoup de tremblements. Cet horaire correspond à une prise de médicament, mais trop tardive. A 20h00, on observe un retour au calme. Le point de mesure de 21h30 confirme l'effet calmant.

Dans un autre exemple, il est également possible, sur la base de l'analyse d'un diagramme de mesure tel qu'illustré à la figure 3, d'ajuster le traitement médicamenteux de l'individu. Par exemple, un ajustement du traitement médicamenteux peut être prescrit par un docteur si la majorité des points correspondants aux mesures du biomarqueur pendant la période temporelle [tᵢ,t_{f}] sont situés dans la deuxième zone du diagramme de référence prédéterminé précédemment décrite. Autrement dit, une telle répartition des points de mesure montre que l'individu présente de forts symptômes et probablement que son traitement n'est plus adapté.

Dans un autre exemple, il est également possible d'effectuer un suivi sur une période temporelle [ti,tf] relativement longue, par exemple, pendant plusieurs mois, de l'évolution de l'état de santé d'un patient. Par exemple, plusieurs diagrammes de mesure peuvent être récoltés à intervalles réguliers et comparés entre eux.

Un autre aspect de l'invention porte sur un produit programme d'ordinateur comportant des instructions pour mettre en oeuvre les étapes du procédé pour surveiller une évolution temporelle pendant une période temporelle [ti,tf], d'un état de santé d'un individu atteint d'une maladie neurodégénérative. Par exemple, les instructions font partie du code de programme informatique 70 stockée dans la mémoire 20 du dispositif 1.

Le produit programme d'ordinateur met notamment en oeuvre les étapes E0, E0a, E1, E2 et E3.

## Revendications

1. Dispositif pour surveiller une évolution temporelle, pendant une période temporelle [ti,tf], d'un état de santé d'un individu atteint d'une maladie neurodégénérative, comprenant :
- au moins une interface d'entrée configurée pour :
- recevoir au moins une séquence temporelle de mesures d'un biomarqueur représentatif d'un symptôme de ladite maladie neurodégénérative, lesdites mesures étant acquises par au moins un capteur ;
- recevoir un diagramme de référence prédéterminé ;
- au moins une unité de traitement configurée pour :
- calculer, pour chaque mesure, un couple de valeurs formé d'une première valeur et d'une deuxième valeur,
- calculer, pour chaque couple de valeurs associé à une mesure de ladite séquence temporelle, une position correspondante sur ledit diagramme de référence prédéterminé,
- déterminer un diagramme de mesure comprenant une superposition du diagramme de référence prédéterminé et desdites positions correspondantes,
- au moins une interface de sortie configurée pour fournir le diagramme de mesure,
l'ensemble des positions correspondantes calculées formant sur le diagramme de mesure une trajectoire représentative de l'évolution temporelle dudit état de santé pendant ladite période temporelle [tᵢ,t_{f}].

2. Dispositif selon la revendication 1, **caractérisé en ce que** la maladie neurodégénérative est l'une parmi la maladie de Parkinson, la sclérose en plaques, ou la maladie de Huntington.

3. Dispositif selon l'une des revendications précédentes, **caractérisé en ce que** ledit biomarqueur est choisi parmi :
- un biomarqueur du tremblement représentatif de tremblements d'au moins une partie du corps,
- un biomarqueur de phonation représentatif de la stabilité de la phonation, et
- un biomarqueur de justesse représentatif de la justesse du geste lors de la calligraphie.

4. Dispositif selon l'une des revendications précédentes, **caractérisé en ce que** l'au moins un capteur est choisi parmi : un accéléromètre, un microphone, un écran tactile un ordiphone.

5. Dispositif selon l'une des revendications précédentes, **caractérisé en ce que** :
- la maladie neurodégénérative est la maladie de Parkinson et le symptôme est un tremblement d'une main de l'individu,
- le diagramme de référence prédéterminé est un diagramme bidimensionnel comprenant un ensemble de points de référence de densité donnée, avec un axe d'abscisses représentant une fréquence de tremblement, et un axe d'ordonnées représentant une intensité de tremblement,
- pour chaque mesure de la séquence temporelle, la première valeur est une fréquence de tremblement et la deuxième valeur est une intensité de tremblement normalisée.

6. Dispositif selon la revendication 5, **caractérisé en ce que** le diagramme de référence prédéterminé comprend une ligne de référence fixe, nommée ligne de crête, au voisinage de laquelle sont localisées les positions correspondantes calculées.

7. Dispositif selon l'une des revendications précédentes, **caractérisé en ce que** le diagramme de référence prédéterminé a été préalablement obtenu par une campagne de mesures temporelles dudit biomarqueur sur un sujet de référence atteint de ladite maladie neurodégénérative pendant un intervalle de temps prédéfini.

8. Dispositif selon la revendication 6 ou les revendications 6 et 7, **caractérisé en ce que** le diagramme de référence prédéterminé comprend, dans un premier voisinage de la ligne de crête, une première zone représentative d'un premier état de santé, et, dans un deuxième voisinage de la ligne de crête distinct du premier voisinage, une deuxième zone représentative d'un deuxième état de santé, le premier état de santé étant dégradé par rapport au deuxième état de santé.

9. Dispositif selon l'une des revendications précédentes, **caractérisé en ce que** l'au moins un capteur est une centrale inertielle intégrée dans une montre connectée ou dans un ordiphone.

10. Procédé mis en oeuvre par ordinateur pour surveiller une évolution temporelle, pendant une période temporelle [ti,tf], d'un état de santé d'un individu atteint d'une maladie neurodégénérative, comprenant :
- recevoir (E0) au moins une séquence temporelle de mesures d'un biomarqueur représentatif d'un symptôme de ladite maladie neurodégénérative pendant ladite période temporelle [ti,tf], lesdites mesures étant acquises par au moins un capteur,
- recevoir (E0a) un diagramme de référence prédéterminé,
- calculer (E1), pour chaque mesure, un couple de valeurs formé d'une première valeur et d'une deuxième valeur,
- calculer (E2), pour chaque couple de valeurs associé à une mesure de la séquence temporelle, une position correspondante sur ledit diagramme de référence prédéterminé,
- déterminer (E3) un diagramme de mesure comprenant une superposition du diagramme de référence prédéterminé et desdites positions correspondantes, l'ensemble des positions correspondantes calculées formant sur le diagramme de mesure une trajectoire représentative de l'évolution temporelle dudit état de santé pendant ladite période temporelle [ti,tf].

11. Procédé selon la revendication 10 mis en oeuvre par un dispositif selon l'une des revendication 1 à 9.

12. Procédé selon l'une des revendications 10 ou 11, mis en oeuvre par un dispositif selon la revendication 8, comprenant en outre :
- afficher ledit diagramme de mesure,
- déterminer un premier ensemble de positions parmi l'ensemble des positions calculées compris dans la première zone,
- identifier, sur la base dudit premier ensemble de positions, un ensemble d'instants nommés instants dégradés, pendant la période temporelle [ti,tf], durant lesquels l'individu était dans le premier état de santé.

13. Procédé selon l'une des revendications 10 ou 11, mis en oeuvre par un dispositif selon la revendication 8, comprenant en outre :
- afficher ledit diagramme de mesure,
- déterminer un premier ensemble de positions parmi l'ensemble des positions calculées compris dans la première zone,
- déterminer un deuxième ensemble de positions parmi l'ensemble des positions calculées compris dans la deuxième zone,
- déduire du premier ensemble de positions et du deuxième ensemble de positions un état de santé global du patient pendant la période temporelle [tᵢ,t_{f}].

14. Produit programme informatique comportant des instructions pour mettre en oeuvre des étapes du procédé selon l'une des revendications 10 à 13, lorsque ce programme est exécuté par un processeur.
